# EUROPEAN PATENT SPECIFICATION

(11) **EP 3 055 410 B1**
(45) Date of publication and mention of the grant of the patent: **06.11.2019**
(21) Application number: 14898951.0
(22) Date of filing: 29.09.2014
(51) Int. Cl.: C12N 5/079, C12N 5/0793, A61K 35/12, A61P 25/28, A61P 25/16, A61P 25/00, A61K 31/436, A61K 45/06, A61K 31/203, A61K 31/366, A61K 31/506, A61K 31/5377, A61K 35/30

(54) **MEDIUM FOR PREPARING A NEURAL CELL AND USAGE THEREOF**
MEDIUM ZUR PRÄPARATION EINES NEURONALEN ZELLEN UND VERWENDUNG DAVON
MILIEU POUR LA PRÉPARATION D'UNE CELLULE NEURONALE ET SON UTILISATION

(30) Priority: 29.07.2014 CN 201410367556
(43) Date of publication of application: 17.08.2016
(73) Proprietor: Shenzhen Cell Inspire Biotechnology Co., Ltd., Shenzhen, Guangdong 518100 (CN)
(72) Inventor: LU, Xiaohua, Shenzhen Guangdong 518100 (CN); YANG, Jiayin, Shenzhen Guangdong 518100 (CN); YANG, Bo, Shenzhen Guangdong 518100 (CN)
(74) Representative: Rössler, Matthias
(86) International application number: PCT/CN2014/087828
(87) International publication number: WO 2016/015378

(56) References cited:
- WO-A1-2012/096705
- WO-A1-2013/127293
- WO-A1-2015/131788
- WO-A2-2010/033906
- WO-A2-2012/022725
- KR-A- 20130 085 767
- METHICHIT WATTANAPANITCH ET AL: "Dual Small-Molecule Targeting of SMAD Signaling Stimulates Human Induced Pluripotent Stem Cells toward Neural Lineages", PLOS ONE, vol. 9, no. 9, 10 September 2014 (2014-09-10), page e106952, XP055353416, DOI: 10.1371/journal.pone.0106952
- JULIA LADEWIG ET AL: "Small molecules enable highly efficient neuronal conversion of human fibroblasts", NATURE METHODS, vol. 9, no. 6, 30 June 2012 (2012-06-30), pages 575-578, XP055345538, ISSN: 1548-7091, DOI: 10.1038/nmeth.1972
- WANG CHIH-HSIANG ET AL: "The role of RhoA kinase inhibition in human placenta-derived multipotent cells on neural phenotype and cell survival", BIOMATERIALS, ELSEVIER SCIENCE PUBLISHERS BV., BARKING, GB, vol. 34, no. 13, 12 February 2013 (2013-02-12), pages 3223-3230, XP028984683, ISSN: 0142-9612, DOI: 10.1016/J.BIOMATERIALS.2012.12.034
- SUJOY K. DHARA ET AL: "Neural differentiation of human embryonic stem cells", JOURNAL OF CELLULAR BIOCHEMISTRY, vol. 105, no. 3, 15 October 2008 (2008-10-15), pages 633-640, XP055353989, US ISSN: 0730-2312, DOI: 10.1002/jcb.21891
- DA-WOON JUNG ET AL: "Reprogram or Reboot: Small Molecule Approaches for the Production of Induced Pluripotent Stem Cells and Direct Cell Reprogramming", ACS CHEMICAL BIOLOGY, vol. 9, no. 1, 17 January 2014 (2014-01-17), pages 80-95, XP055111768, ISSN: 1554-8929, DOI: 10.1021/cb400754f

## Description

### FIELD

The present disclosure relates to biomedical field, particularly to a method for preparing an oligodendrocyte progenitor cell.

### BACKGROUND

Central nervous system (CNS) disorders, such as Alzheimer's Disease (AD), Parkinsin's Disease (PD), Amyotrophic Lateral Sclerosis (ALS), Multiple Sclerosis (MS), and Leukodystrophies, affect millions of people worldwide and most of CNS diseases currently have no cure. Transplantation of neural cells has been demonstrated in non-human primates and rodents to be a very promising therapeutic strategy for treating CNS disorders. However, cell therapy requires high quality and large quantities of primary neural cells that are not readily obtainable. Different methods have been developed during last two decades to produce neural cells *in vitro.* These technologies include generation of neural cells from human embryonic stem cells (hESC), from induced human pluripotent stem cells (hiPSC), and from direct conversion of somatic cells. There are several limitations to existing art. For example, neural cells derived from hESC encounter the safety issue due to potential tumorgenesis caused by residual hESC in the products as well as immune rejection issue due to allogeneic transplantation. hiPSC can be obtained from specific patients and thus alleviate the immunorejection issue, but like hESC, it still encounters the safety issue.

Recently, the field of direct somatic lineage conversion has attracted much attention. In 2010, Wernig's group first demonstrated that a set of transcription factors can convert fibroblasts into neurons. Several laboratories have used various neural factors and microRNAs to generate fibroblast-neuron conversion. Very recently, it was reported that over-expression transcription factors can mediate reprogramming of mouse fibroblasts to myelinogenic OPCs. Thus, direct fibroblast-OPC conversion provides an alternative, potentially complementary, tool to many of the proposed applications of hESC/hiPSC technology for both disease modeling and development of cell-based therapies. Direct conversion or reprogramming has a number of advantages, including: the time required to generate, expand and differentiate pluripotent cells is avoided, and the postmitotic state of induced neural cells has a much lower risk of cancer and teratoma formation. Therefore, neural cells derived from direct conversion of fibroblasts have been favored for autologous transplantation. However, all reports on direct conversion of somatic cells into neural cells have been using transcription factors, which often involve lengthy conversion procedure and are regarded not safe due to integration of viral vector sequence into the genome.

### SUMMARY

The present disclosure directs to solve at least one of the problems existing in the prior art. Therefore, one purpose of present disclosure is to provide a method for efficiently preparing oligodendrocyte progenitor cells with high safety and suitable for autotransplantation using protein kinase inhibitor.

In one aspect of present disclosure, a protein kinase inhibitor for use in preparing a oligodendrocyte progenitor cell from a fibroblast cell is provided. The inventors have surprisingly found that in the presence of protein kinase inhibitors, it is not necessary to use transcription factors, and fibroblast cells can be efficiently transformed into oligodendrocyte progenitor cells.

As described in the present disclosure, the protein kinase inhibitor maybe one selected from ROCK inhibitor, AKT/mTOR inhibitor, PI3K inhibitor, FAK inhibitor and immunosuppresants. Thereby, it is possible to effectively induce non-neural cells to transform into neural cells.

According to embodiments of present disclosure, the protein kinase inhibitor is at least one selected from ROCK inhibitor Y27632, AKT/mTOR inhibitor Palomid 529, PI3K inhibitor LY294002, FAK inhibitor PF562271 and immunosuppresants rapamycin. Thereby, it is possible to effectively induce non-neural cells to transform into neural cells with high convention efficiency.

Thereby, the prepared oligodendrocyte progenitor cells can be effectively used for autologous transplantation, treatment of central nervous system disorders and spinal cord injury with high safety while without immune rejection.

In one aspect of present disclosure, a method for preparing an oligodendrocyte progenitor cell is provided, and according to embodiments of present disclosure, the method may comprise: culturing a fibroblast cell in the present of a protein kinase inhibitor. The inventors have found that by the method of present disclosure, the oligodendrocyte progenitor cells can be prepared rapidly and efficiently without requirement of the transcription factors. The prepared oligodendrocyte progenitor cells are suitable for autologous transplantation, treatment of central nervous system disorders and spinal cord injury with high safety while without immune rejection.

As described in the present disclosure, the protein kinase inhibitor maybe selected from ROCK inhibitor, AKT/mTOR inhibitor, PI3K inhibitor, FAK inhibitor and immunosuppresants. Thereby, it is possible to effectively induce non-neural cells to transform into neural cells.

According to embodiments of present disclosure, the protein kinase inhibitor is at least one selected from ROCK inhibitor Y27632, AKT/mTOR inhibitor Palomid 529, PI3K inhibitor LY294002, FAK inhibitor PF562271 and immunosuppresants rapamycin. Thereby, it is possible to effectively induce fibroblast cells to convert into oligodendrocyte progenitor cells with high convention efficiency.

The application further describes a kit, the kit described herewith may comprise: a first medium, the first medium may comprise: a first basic medium, a protein kinase inhibitor, brain-like neurotrophic factor (BDNF), neurotrophic factor 3 (NT3), sodium valproate (VPA), dibutyryl cyclic adenosine monophosphate (dbcAMP), and retinoic acid (RA), wherein the protein kinase inhibitor maybe selected from ROCK inhibitor, AKT/mTOR inhibitor, PI3K inhibitor, FAK inhibitor and immunosuppresants.

As described herewith, the kit may further comprises: a second medium, or/and, a third medium, wherein the second medium comprises: a second basic medium, a protein kinase inhibitor, N2 cell culture medium additive (N-2), B27 cell culture medium additives (B-27), glutamine (Glutamax), hedgehog protein (SHH), fibroblast growth factor (FGF2), recombinant human platelet derived growth factor (PDGF-AA), and retinoic acid, or/and a third medium, wherein the third medium comprises: a third basic medium, a protein kinase inhibitor, N2 cell culture medium additive (N-2), B27 cell culture medium additives (B-27), glutamine (Glutamax), hedgehog protein (SHH), noggin protein (Noggin), dibutyryl cyclic adenosine monophosphate, insulin-like growth factor (IGF), neurotrophic factor 3, and retinoic acid, wherein the protein kinase maybe selected from ROCK inhibitor, AKT/mTOR inhibitor, PI3K inhibitor, FAK inhibitor and immunosuppresants. Thereby, it is possible to effectively prepare mature different types of nerual cells.

According to embodiments of present disclosure, the first basic medium is neuronal medium, the second basic medium is DMEM/F12 medium, and the third basic medium is DMEM/F12 medium. Thereby, it is advantageous to convert non-neural cells into neural cells.

According to embodiments of present disclosure, the protein kinase inhibitor is at least one selected from ROCK inhibitor Y27632, AKT/mTOR inhibitor Palomid 529, PI3K inhibitor LY294002, FAK inhibitor FPF562271 and immunosuppresants rapamycin. Preferably he protein kinase inhibitor is ROCK inhibitor Y27632.

According to embodiments of present disclosure, the first medium may comprise: 5µM - 20µM Y27632, 5 ng/ml - 20 ng/ml BDNF, 20 ng/ml - 80 ng/ml NT3, 0.5 mM - 1.5 mM VPA, 25µM -100 µM dbcAMP, and 0.5µM - 1µM retinoic acid; the second medium may comprise: 5µM - 20µM Y27632, 1x N-2, 1x B-27, 1x Glutamax, 100 ng/ml - 400 ng/ml SHH, 10 ng/ml - 40 ng/ml FGF2, 10 ng/ml - 40 ng/ml PDGF-AA, and 0.5µM - 1µM retinoic acid; the third medium may comprise: 5µM - 20µM Y27632, 1x N-2, 1x B-27, 1x Glutamax, 100 ng/ml - 400 ng/ml SHH, 50 ng/ml - 200 ng/ml Noggin, 50 ng/ml - 200 ng/ml dbcAMP, 50 ng/ml - 200 ng/ml IGF, 20 ng/ml - 80 ng/ml NT3, and 0.5µM - 1µM retinoic acid. Thereby, the non-neural cell can be directly induced to convert into neural cell rapidly and effectively with high efficiency.

According to embodiments of present disclosure, the first medium may comprise: 10µM Y27632, 10 ng/ml BDNF, 10 ng/ml NT3, 1 mM VPA, 50 µM dbcAMP, and 0.5µM retinoic acid; the second medium may comprise: 10µM Y27632, 1x N-2, 1x B-27, 1x Glutamax, 200 ng/ml SHH, 20 ng/ml FGF2, 20 ng/ml PDGF-AA, and 0.5µM retinoic acid; the third medium may comprise: 10µM Y27632, 1x N-2, 1x B-27, 1x Glutamax, 200 ng/ml SHH, 100 ng/ml Noggin, 100 ng/ml dbcAMP, 100 ng/ml IGF, 10 ng/ml NT3, and 0.5µM retinoic acid. Thereby, the non-neural cell can be directly induced to convert into neural cell rapidly and effectively with high efficiency. The prepared neural cells can be effectively used for autologous transplantation, treatment of central nervous system disorders and spinal cord injury with high safety while without immune rejection.

Further, the kit for use in preparing a neural cell from a non-neural cell is described. The inventors have found that the non-neural cell may be induced to convert into neural cells with the above kit efficiently, and the prepared neural cells may be effectively used for autologous transplantation, treatment of central nervous system disorders and spinal cord injury with high safety while without immune rejection.

In another aspect of present disclosure, a method for preparing an oligodendrocyte progenitor cell is provided, and according to embodiments of present disclosure, the method may comprise:
(1) culturing a fibroblast cell with a first medium,
   wherein the first medium may comprise: a first basic medium, a protein kinase inhibitor, BDNF, NT3, VPA, dbcAMP, and retinoic acid, wherein the protein kinase inhibitor is at least one selected from ROCK inhibitor, AKT/mTOR inhibitor, PI3K inhibitor, FAK inhibitor and immunosuppresants. The inventors have found that by the method of present disclosure, the neural cells can be prepared rapidly and efficiently without requirement of the transcription factors. The prepared neural cells are suitable for autologous transplantation, treatment of central nervous system disorders and spinal cord injury with high safety while without immune rejection.
   According to embodiments of present disclosure, the method for preparing a oligodendrocyte progenitor cell further comprises:
(2) Culturing the cell with a second medium or a third medium after the culturing with the first medium.
   wherein the second medium comprises: a second basic medium, a protein kinase inhibitor, N-2, B-27, Glutamax, SHH, FGF2, PDGF-AA, and retinoic acid, the third medium comprises: a third basic medium, a protein kinase inhibitor, N-2, B-27, Glutamax, SHH, Noggin, dbcAMP, IGF, NT3, and retinoic acid. According to embodiments of present disclosure, the protein kinase inhibitor is selected from ROCK inhibitor, AKT/mTOR inhibitor, PI3K inhibitor, FAK inhibitor and immunosuppresants. By the method of present disclosure, the neural cells can be prepared rapidly and efficienly without requirement of the transcription factors. The prepared oligodendrocyte progenitor cells are suitable for autologous transplantation, treatment of central nervous system disorders and spinal cord injury with high safety while without immune rejection.

According to embodiments of present disclosure, the first basic medium is neuronal medium, the second basic medium is DMEM/F12 medium, the third basic medium is DMEM/F12 medium. Thereby, it is advantageous to convert non-neural cells into neural cells.

According to embodiments of present disclosure, the protein kinase inhibitor is at least one selected from ROCK inhibitor Y27632, AKT/mTOR inhibitor Palomid 529, PI3K inhibitor LY294002, FAK inhibitor PF562271 and immunosuppresants rapamycin. Thereby, the non-neural cell can be induced to convert into neural cell with high convention efficiency.

According to embodiments of present disclosure, the first medium may comprise: 5µM - 20µM Y27632, 5 ng/ml - 20 ng/ml BDNF, 20 ng/ml - 80 ng/ml NT3, 0.5 mM - 1.5 mM VPA, 25µM -100 µM dbcAMP, and 0.5µM - 1µM retinoic acid; the second medium may comprise: 5µM - 20µM Y27632, 1x N-2, 1x B-27, 1x Glutamax, 100 ng/ml - 400 ng/ml SHH, 10 ng/ml - 40 ng/ml FGF2, 10 ng/ml - 40 ng/ml PDGF-AA, and 0.5µM - 1µM retinoic acid; the third medium may comprise: 5µM - 20µM Y27632, 1x N-2, 1x B-27, 1x Glutamax, 100 ng/ml - 400 ng/ml SHH, 50 ng/ml - 200 ng/ml Noggin, 50 ng/ml - 200 ng/ml dbcAMP, 50 ng/ml - 200 ng/ml IGF, 20 ng/ml - 80 ng/ml NT3, and 0.5µM - 1µM retinoic acid. Thereby, the non-neural cell can be directly induced to convert into neural cell rapidly and effectively with high efficiency.

According to embodiments of present disclosure, the first medium may comprise: 10µM Y27632, 10 ng/ml BDNF, 10 ng/ml NT3, 1 mM VPA, 50 µM dbcAMP, and 0.5µM retinoic acid; the second medium may comprise: 10µM Y27632, 1x N-2, 1x B-27, 1x Glutamax, 200 ng/ml SHH, 20 ng/ml FGF2, 20 ng/ml PDGF-AA, and 0.5µM retinoic acid; the third medium may comprise: 10µM Y27632, 1x N-2, 1x B-27, 1x Glutamax, 200 ng/ml SHH, 100 ng/ml Noggin, 100 ng/ml dbcAMP, 100 ng/ml IGF, 10 ng/ml NT3, and 0.5µM retinoic acid. Thereby, the non-neural cell can be directly induced to convert into neural cell rapidly and effectively with high efficiency. The prepared neural cells can be effectively used for autologous transplantation, treatment of central nervous system disorders and spinal cord injury with high safety while without immune rejection.

Further described in this invention is a oligodendrocyte progenitor cell or their derivative. The oligodendrocyte progenitor cell maybe prepared by the method mentioned above. The inventors have found that the oligodendrocyte progenitor cell or their derivative of present disclosure maybe suitable for autologous transplantation, treatment of central nervous system disorders and spinal cord injury with high safety while without immune rejection.

Further described in the present disclosure is the oligodendrocyte progenitor cell or their derivative for use in preparing a drug for the treatment of central nervous system disorder and spinal cord injury is provided.

According to description of present disclosure, the Central nervous system disorder is at least one selected from Alzheimer's Disease, Parkinsin's Disease, Amyotrophic Lateral Sclerosis, Multiple Sclerosis, and Leukodystrophies. Thereby, the treatment effect is better.

### BRIEF DESCRIPTION OF THE DRAWINGS

Fig.1 is a graph showing the immunostaining results of iOPCs according to an embodiment of the present disclosure, in which
Fig.1A is a schematic diagram of the flow of screening process,
Fig.1B are images of cells in the control and experimental groups at 7 days of culture,
Fig.1C is conversion rate in the control and experimental groups at 7 days of culture;
Fig.2 is a graph showing the immunostaining results of iOPCs and iOLs according to example 3 of the present disclosure, in which
Fig.2A shows the immunostaining results of iOPCs before differentiation culture,
Fig.2B shows the immunostaining results of iOLs after differentiation culture;
Fig.3 is a graph showing the results of immunostaining and Western blot according to example 4 of the present disclosure, in which
Fig.3A is the result of the Western blot analysis,
Fig.3B is the immunostaining results of iOPCs,
Fig.3C is the immunostaining results of iOLs;
Fig.4 is a graph showing the results of a microarray analysis according to example 5 of the present disclosure, in which
Fig.4A shows the differential expression profiles of iOPCs, human brain-derived OPCs, iOLs and parental fibroblasts,
Fig.4B shows the results of cluster analysis of differentially expressed genes between iOPCs, human brain-derived OPCs, iOLs and parental fibroblasts;
Fig.5 is a graph showing the result of immuno-histochemical detection according to example 6 of the present disclosure, in which
Fig.5A are images of the immunofluorescence results and H & E staining results for the site of iOPs injection of the same brain tissue sections,
Figure 5B are images of the immunostaining results for the site of iOPCs injection of brain tissue sections,
Figure 5C are confocal microscopic photographs of the iOPCs site of injection of the brain tissue sections;
Fig.6 is a graph showing the immunostaining results of neutral cells converted from IMR90 fibroblasts according to example 7 of the present disclosure,
Fig.7 is a graph showing the electrophysiological examination results neutral cells converted from IMR90 fibroblasts according to example 7 of the present disclosure,
Fig.7a shows current traces (upper panel) recorded in the process in which cells were depolarized by voltage steps from -60 to +60 mV in 10 mV increments and the relationship (lower panel) between the current-voltage (I-V) and sodium current,
Fig.7b shows traces of membrane currents recorded in the process of a voltage ramp from -80mV to +60mV over 500ms,
Fig.7c shows traces of spontaneous synaptic currentsrecorded at a holding potential of -80 mV; and
Fig.8 is a graph showing the immunostaining results of astrocyte cells converted from IMR90 fibroblasts according to example 7 of the present disclosure,

### DETAILED DESCRIPTION

The embodiments of the present invention will be described in detail below. The embodiments described below are exemplary and are intended to be illustrative of the invention and are not to be construed as limiting the invention. The specific technical or conditions not indicated in the examples, are either in accordance with the techniques or conditions described in the literature in the art or in accordance with the product specifications. The reagents or instruments used not indicate the manufacturer are available through the purchase of conventional products.

In one aspect of present disclosure, a protein kinase inhibitor for use in preparing a oligodendrocyte progenitor cell from a fibroblast cell is provided. Inventors found that, the protein kinase inhibitor is able to convert the fibroblast cell into the oligodendrocyte progenitor cell efficiently, without using any transcription factor.

According to embodiments of present disclosure, the protein kinase inhibitor is at least one selected from ROCK inhibitor Y27632, AKT/mTOR inhibitor Palomid 529, PI3K inhibitor LY294002, FAK inhibitor PF562271 and immunosuppresants rapamycin.The protein kinase inhibitor is sufficient to reprogram the non-neural cell into the neural cell.

In a second broad aspect of present disclosure, a method for preparing a oligodendrocyte progenitor cell is provided, and according to embodiments of present disclosure, the method may comprise: culturing a fibroblast cell in the present of a protein kinase inhibitor. The inventors have found that by the method of present disclosure, the oligodendrocyte progenitor cells can be prepared rapidly and efficiently without requirement of the transcription factors. The prepared oligodendrocyte progenitor cells are suitable for autologous transplantation, treatment of central nervous system disorders and spinal cord injury with high safety while without immune rejection.

As described in the present disclosure, the protein kinase inhibitor maybe one selected from ROCK inhibitor, AKT/mTOR inhibitor, PI3K inhibitor, FAK inhibitor and immunosuppresants. Using the protein kinase inhibitor can convert the non-neural cell into the neural cell efficiently.

According to embodiments of present disclosure, the protein kinase inhibitor is at least one selected from ROCK inhibitor Y27632, AKT/mTOR inhibitor Palomid 529, PI3K inhibitor LY294002, FAK inhibitor PF562271 and immunosuppresants rapamycin. The protein kinase inhibitor is sufficient to reprogram the non-neural cell into the neural cell.

Further described in the present invention is a kit, the kit may comprise: a first medium, the first medium may comprise: a first basic medium, a protein kinase inhibitor, BDNF, NT3, VPA, dbcAMP, and retinoic acid, according to embodiments of present disclosure, the protein kinase inhibitor is at least one selected from ROCK inhibitor, AKT/mTOR inhibitor, PI3K inhibitor, FAK inhibitor and immunosuppresants. Using the kit of present disclosure, the non-neural cell can be induced into the neural cell directly with high efficiency and convenience.

According to the description, the first basic medium is neuronal medium.

According to the description, the first medium may comprise: 5µM - 20µM Y27632, 5 ng/ml - 20 ng/ml BDNF, 20 ng/ml - 80 ng/ml NT3, 0.5 mM - 1.5 mM VPA, 25µM -100 µM dbcAMP, and 0.5µM - 1µM retinoic acid. Thereby, the non-neural cell can be directly induced to convert into neural cell rapidly and effectively with high efficiency.

According to the description, the first medium may comprise: 10µM Y27632, 10 ng/ml BDNF, 10 ng/ml NT3, 1 mM VPA, 50 µM dbcAMP, and 0.5µM retinoic acid. Thereby, the non-neural cell can be directly induced to convert into neural cell rapidly and effectively with high efficiency. The prepared neural cells can be effectively used for autologous transplantation, treatment of central nervous system disorders and spinal cord injury with high safety while without immune rejection.

According to the description, the kit may future comprises: a second medium, or/and, a third medium. According to embodiments of present disclosure, the second medium comprises: a second basic medium, a protein kinase inhibitor, N-2, B-27, Glutamax, SHH, FGF2, PDGF-AA, and retinoic acid, or/and a third medium. The third medium comprises: a third basic medium, a protein kinase inhibitor, N-2, B-27, Glutamax, SHH, Noggin, dbcAMP, IGF, NT3, and retinoic acid. According to embodiments of present disclosure, the protein kinase inhibitor is at least one selected from ROCK inhibitor, AKT/mTOR inhibitor, PI3K inhibitor, FAK inhibitor and immunosuppresants. Thereby, it is possible to effectively prepare mature different types of nerual cells.

According to the description, the neural cell maybe one selected from oligodendrocyte progenitor cell, mature oligodendrocyte, neuron and astrocyte. Thereby, the prepared neural cells may be effectively used for autologous transplantation, treatment of central nervous system disorders and spinal cord injury with high safety while without immune rejection.

According to the description, the second basic medium is DMEM/F12 medium, the third basic medium is DMEM/F12 medium. The basic mediums mentioned above are in favor of the conversion from the non-neural cell to the neural cell.

According to the description, the protein kinase inhibitor is at least one selected from of Y27632, Palomid 529, LY294002, PF562271 and rapamycin. The protein kinase inhibitor is sufficient to reprogram the non-neural cell into the neural cell.

According to the description, the second medium may comprise: 5µM - 20µM Y27632, 1x N-2, 1x B-27, 1x Glutamax, 100 ng/ml - 400 ng/ml SHH, 10 ng/ml - 40 ng/ml FGF2, 10 ng/ml - 40 ng/ml PDGF-AA, and 0.5µM - 1µM retinoic acid; the third medium may comprise: 5µM - 20µM Y27632, 1x N-2, 1x B-27, 1x Glutamax, 100 ng/ml - 400 ng/ml SHH, 50 ng/ml - 200 ng/ml Noggin, 50 ng/ml - 200 ng/ml dbcAMP, 50 ng/ml - 200 ng/ml IGF, 20 ng/ml - 80 ng/ml NT3, and 0.5µM - 1µM retinoic acid. Thus, the non-neural cell will convert into the neural cell quickly and efficiently when cultured with the medium mentioned above, and the conversion rate is very high.

According to the description, the second medium may comprise: 10µM Y27632, 1x N-2, 1x B-27, 1x Glutamax, 200 ng/ml SHH, 20 ng/ml FGF2, 20 ng/ml PDGF-AA, and 0.5µM retinoic acid; the third medium may comprise: 10µM Y27632, 1x N-2, 1x B-27, 1x Glutamax, 200 ng/ml SHH, 100 ng/ml Noggin, 100 ng/ml dbcAMP, 100 ng/ml IGF, 10 ng/ml NT3, and 0.5µM retinoic acid. Thereby, the non-neural cell can be directly induced to convert into neural cell rapidly and effectively with high efficiency. The prepared neural cells may be effectively used for autologous transplantation, treatment of central nervous system disorders and spinal cord injury with high safety while without immune rejection.

Further described is the kit for use in preparing a neural cell. Using the kit as described above, the non-neural cell may be induced to convert into neural cells efficiently, and the neural cell maybe suitable for autologous transplantation to treat central nervous system disorder and spinal cord injury with high safety, and there is no immune rejection.

In a further aspect of present disclosure, a method for preparing a oligodendrocyte progenitor cell is provided, and according to embodiments of present disclosure, the method may comprise:
(1) Culturing a fibroblast cell with a first medium,
   According to embodiments of present disclosure, the first medium may comprise: a first basic medium, a protein kinase inhibitor, BDNF, NT3, VPA, dbcAMP, and retinoic acid.
   According to embodiments of the present invention, the type of the first basic medium is not particularly limited, and can be advantageously selected by a person skilled in the art, as long as it can facilitate the induction of non-neuronal cells into neutral cells. According to an embodiment of the present invention, the first basic medium is neuronal medium. Thus, it is advantageous to convert non-neuronal cells into neutral cells.
   According to embodiments of present disclosure, the protein kinase inhibitor is at least one selected from Y27632, Palomid 529, LY294002, PF562271 and rapamycin, preferably is Y27632. Thereby the non-neural cell, which is a fibroblast cell, can directly convert into the neural cell, which is oligodendrocyte progenitor cell, with high efficiency and convention rate.
   According to embodiments of present disclosure, the first medium may comprise: 5µM - 20µM Y27632, 5 ng/ml - 20 ng/ml BDNF, 20 ng/ml - 80 ng/ml NT3, 0.5 mM - 1.5 mM VPA, 25µM -100 µM dbcAMP, and 0.5µM - 1µM retinoic acid. Thereby, the non-neural cell can be directly induced to convert into neural cell rapidly and effectively with high efficiency. If the concentration of each component is too high or too low, the conversion effect is not ideal.
   According to embodiments of present disclosure, the first medium may comprise: 10µM Y27632, 10 ng/ml BDNF, 10 ng/ml NT3, 1 mM VPA, 50 µM dbcAMP, and 0.5µM retinoic acid. Thereby, the non-neural cell, which is fibroblast cell, can be gradually induced to convert into neural cell, which is oligodendrocyte progenitor cell, under most suitable conditions with high efficiency. The prepared oligodendrocyte progenitor cells can be effectively used for autologous transplantation, treatment of central nervous system disorders and spinal cord injury with high safety while without immune rejection.
   As described in the present disclosure, the non-neural cell is human cell. The human cell maybe one selected from fibroblast cell, epithelial cell, adult cell and neonatal cell. Thus, the non-neural cell, which is fibroblast cell, can convert into the neural cell, which is oligodendrocyte progenitor cell, efficiently in the present of the protein kinase inhibitor.
(2) Culturing the cell with a second medium or a third medium after the culturing with the first medium.

According to embodiments of present disclosure, the second medium comprises: a second basic medium, a protein kinase inhibitor, N-2, B-27, Glutamax, SHH, FGF2, PDGF-AA, and retinoic acid, the third medium comprises: a third basic medium, a protein kinase inhibitor, N-2, B-27, Glutamax, SHH, Noggin, dbcAMP, IGF, NT3, and retinoic acid. Thereby, the non-neural cell, which is fibroblast cell, can be gradually induced to convert into neural cell, which is oligodendrocyte progenitor cell, under most suitable conditions with high efficiency. The prepared neural cells, which are oligodendrocyte progenitor cells, can be effectively used for autologous transplantation, treatment of central nervous system disorders and spinal cord injury with high safety while without immune rejection.

According to embodiments of the present invention, the kind of the second basic medium is not particularly limited, and one skilled in the art can select it flexibly according to the actual situation. According to an embodiment of the present invention, the second basic medium is DMEM / F12 medium.

According to embodiments of present disclosure, the protein kinase inhibitor is at least one selected from Y27632, Palomid 529, LY294002, PF562271 and rapamycin, preferably is Y27632. The protein kinase inhibitor is sufficient to reprogram the non-neural cell into the neural cell.

According to embodiments of present disclosure, the second medium may comprise: 5µM - 20µM ROCK inhibitor (Y27632), 1x N-2, 1x B-27, 1x Glutamax, 100 ng/ml - 400 ng/ml SHH, 10 ng/ml - 40 ng/ml FGF2, 10 ng/ml - 40 ng/ml PDGF-AA, and 0.5µM - 1µM retinoic acid. Thereby, the non-neural cell can be directly induced to convert into neural cell rapidly and effectively with high efficiency.

According to embodiments of present disclosure, the second medium may comprise: 10µM Y27632, 1x N-2, 1x B-27, 1x Glutamax, 200 ng/ml SHH, 20 ng/ml FGF2, 20 ng/ml PDGF-AA, and 0.5µM retinoic acid. Thereby, the non-neural cell can be gradually induced to convert into neural cell under most suitable conditions with high efficiency. The prepared neural cells can be effectively used for autologous transplantation, treatment of central nervous system disorders and spinal cord injury with high safety while without immune rejection.

According to embodiments of present disclosure, the third medium comprises: a third basic medium, a protein kinase inhibitor, N-2, B-27, Glutamax, SHH, Noggin, dbcAMP, IGF, NT3, and retinoic acid.

According to embodiments of the present invention, the kind of the third basic medium is not particularly limited, and one skilled in the art can select it flexibly according to the actual situation. According to an embodiment of the present invention, the third basic medium is DMEM / F12 medium.

According to embodiments of present disclosure, the protein kinase inhibitor is at least one selected from Y27632, Palomid 529, LY294002, PF562271 and rapamycin, preferably is Y27632. Thereby the non-neural cell can directly convert into the neural cell with high efficiency and convention rate.

According to embodiments of present disclosure, the third medium may comprise: 5µM - 20µM ROCK inhibitor (Y27632), 1x N-2, 1x B-27, 1x Glutamax, 100 ng/ml - 400 ng/ml SHH, 50 ng/ml - 200 ng/ml Noggin, 50 ng/ml - 200 ng/ml dbcAMP, 50 ng/ml - 200 ng/ml IGF, 20 ng/ml - 80 ng/ml NT3, and 0.5µM - 1µM retinoic acid. Thus, the non-neural cell will convert into the neural cell quickly and efficiently when cultured with the medium mentioned above, and the conversion rate is very high.

According to embodiments of present disclosure, the third medium may comprise: 10µM Y27632, 1x N-2, 1x B-27, 1x Glutamax, 200 ng/ml SHH, 100 ng/ml Noggin, 100 ng/ml dbcAMP, 100 ng/ml IGF, 10 ng/ml NT3, and 0.5µM retinoic acid. Thus, the non-neural cell will convert gradually into the neural cell quickly and efficiently when cultured with the medium mentioned above under most suitable conditions, and the conversion rate is very high. Besides, the neural cell is suitable for autologous transplantation to treat central nervous system disorder and spinal cord injury with high safety, and there is no immune rejection.

As described herewith, the neural cell maybe one selected from oligodendrocyte progenitor cell, mature oligodendrocyte, neuron and astrocyte. Thereby, the prepared neural cells may be effectively used for autologous transplantation, treatment of central nervous system disorders and spinal cord injury with high safety while without immune rejection.

By the method of present disclosure, the neural cells, which are oligodendrocyte progenitor cells, can be prepared rapidly and efficiently without requirement of the transcription factors. The prepared oligodendrocyte progenitor cells are suitable for autologous transplantation, treatment of central nervous system disorders and spinal cord injury with high safety while without immune rejection.

Further described is a neural cell or their derivative, the neural cell maybe prepared by the method mentioned above. The inventors have found that the neural cell or their derivative described herewith are suitable for autologous transplantation, treatment of central nervous system disorders and spinal cord injury with high safety while without immune rejection.

Further described is the neural cell or their derivative for use in preparing a drug for the treatment of central nervous system disorder and spinal cord injury.

According to the description, the type of the central nervous system disorder is not particularly limited. According to the description, the aforementioned medicament maybe suitable for use in the treatment of the central nervous system disorder selected from Alzheimer's Disease, Parkinsin's Disease, Amyotrophic Lateral Sclerosis, Multiple Sclerosis, and Leukodystrophies. Thereby, the treatment effect is better.

### EXAMPLE 1: screening of protein kinase inhibitor library

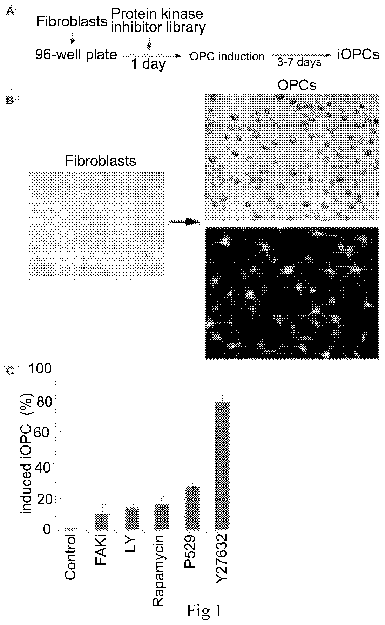

Coated cell culture 24-well plate with 0.1 mg/ml Poly-L-ornithine solution (sigma, P4957) for at least 3 hours. Then washed wells 3 times with autoclaved MilliQ water, 5 mins per time. Added 500ul/well of 2 µg/ml Fibronectin (sigma, F0556) and 10 µg/ml Laminin (ROCHE, 11243217001) solution in IX PBS and placed in incubator overnight to coat. Aspirated the Fibronectin/Laminin solution from the wells, washed with culture medium. Plated fibroblast cells with Basic Medium at 2x10^4 cells (human adult dermal fibroblasts, ATCC, PCS-201-012) per well, cultured overnight. The Basic Medium comprises: DMEM high glucose (HyClone, SH30022), 10% FBS (Fisher Scientific, SH3007003), 10 mM HEPES (CORNING, 25060-CL), 1x MEM NEAA (100X) (GIBCO, 11140) (MEM Non-Essential Amino Acids Solution), 1 mM Na Pyruvate (GIBCO, 25000), 1x 2-Mercaptoethanol (1000X) (GIBCO, 21985), 1x Glutamax (100X) (GIBCO, 35050), penicillin/streptomycin. Aspirated Basic Medium from the wells. Washed with PBS once. Fed wells with Induction Medium. The Induction Medium comprises: Neuronal Medium (ScienCell, 1521), 10 ng/ml BDNF (PROSPEC, CYT-207) (Brain-Derived Neurotrophic Factor), 10 ng/ml NT3 (PROSPEC, CYT-257) (neurotrophin 3), 1 mM VPA (sigma, P4543) (Valproic acid sodium salt), 50 µM dbcAMP (sigma, D0627) (Dibutyryl cAMP sodium salt), penicillin/streptomycin, 0.5 µM RA (Retinoic acid) (sigma, R2625), 2 µM protein inhibitor (protein kinase inhibitor library, Library I ,II, III: 240 inhibitors, Calbiochem, Cat# 539744,539745 & 539746). The Induction Medium without protein kinase inhibitor was added to the control group. Changed medium every two days. The morphology of the cells was observed during the culture and the conversion rate was calculated at day 7 (conversion rate = number of induced neural cells / number of total cells). The results of several screening experiments are shown in Fig.1. Fig.1a is a schematic diagram of the flow of screening process. Fig.1b are images of cells in the control and experimental groups at 7 days of culture, wherein the left panel is image of cells in control group, while the right panel is the image of cells in experimental group in which ROCK inhibitor Y27632 (Rock inhibitors Y-27632 and Y-27632 were used interchangeably herein) was added. Fig.1c is the result of conversion rate in the control and experimental groups in which different protein inhibitors was add at 7 days of culture. Control represent control group.

When cells changed their morphology into neural-like cells (about 7 days from fibroblasts seeding), cells were cultured with OPC (oligodendrocyte precursor cells) medium (DMEM/F12,Invitrogen, 11320), 1x N-2 (R&D Systems), 1x B-27 without vitamin A (Invitrogen), 1x Glutamax (GIBCO, 35050), 200 ng/ml SHH (R&D Systems), 20 ng/ml FGF2 (R&D Systems), 20 ng/ml PDGF-AA (R&D Systems), penicillin/streptomycin, 10 µM Y-27632 (Enzo Life Sciences, ALX-270-333-M005), 0.5 µM RA (Sigma, R2625) for induction of OPCs.

As shown in Fig.1, addition of ROCK inhibitors Y-27632 can induce human adult dermal fibroblasts to convert into neural cells with high efficiency, whereas no conversion of fibroblasts to neural cells was observed in control group without kinase inhibitor. In particular, inventors observed that a subpopulation of fibroblasts underwent a marked morphological change in only 3-7 days in neural induction medium with kinase inhibitor treatments, from large, flat, spindle shaped cells (fibroblasts) to small, bi- of multi-polar cells, termed iOPCs (right panel in Fig. 1b). As can be seen from Fig.1c, when the culture medium containing ROCK inhibitor Y27632 was cultured into fibroblasts, the conversion rate was the highest, reaching about 80%. After the initial screening, inventors selected the top 5 candidate inhibitors with the highest conversation rates, namely the ROCK inhibitor Y27632 (Y27632), the AKT / mTOR inhibitor Palomid 529 (P529), the PI3K inhibitor LY294002 (LY), the FAK inhibitor PF562271 (FAKi), and Immunosuppressant rapamycin for further analysis.

### EXAMPLE 2:

Using the same method with EXAMPLE 1, fiber cell line IMR90 and WI38 were induced ROCK inhibitors Y27632, Palomid 529, LY294002, PF562271 or rapamycin. The result showed that: cultured for a period of time with neural induction medium including the protein kinase inhibitor, in both cell lines, fibroblasts were converted into oligodendrocyte precursor cells. Results of the IMR90 cells induced by the ROCK inhibitor Y27632 and the conversion results are shown in Figure 1.

### EXAMPLE 3:

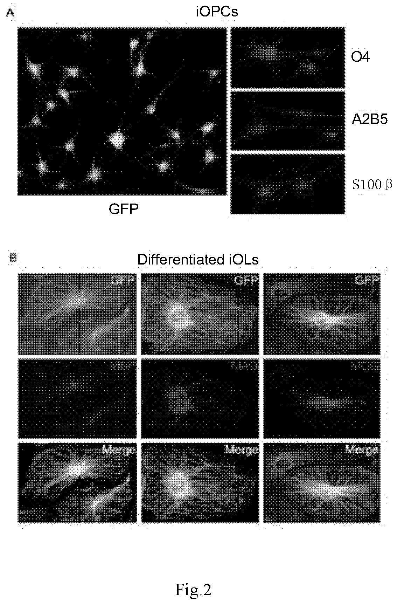

In this example, whether the iOPCs prepared by inducing can be differentiated into mature oligodendrocytes can be assessed as follows:
Using mature medium (10 µM of ROCK inhibitor Y27632 (Enzo Life Sciences, ALX-270-333-M005) with thyroid hormone (a known oligodendrocyte differentiation inducer) while without growth factor , 1x N2 cell culture medium additive, 1x B27 cell culture additive, 1x glutamine, 200 ng / ml hedgehog protein, 100 ng / ml Noggin (R & D systems), 100 ng / ml dibutyryl cyclic adenosine (Sigma, D0627), 100 ng / ml insulin-like growth factor (R & D systems), 10 ng / ml neurotrophic factor 3 (PROSPEC, CYT-257), and 0.5 µM retinoic acid (sigma, R2625)) to differentiated culture iOPCs obtained after 7 days in a neuronal induction medium containing 2 µM of ROCK inhibitor Y27632 in Example 1 to obtain iOLs (induced oligodendrocytes). iOPCs and iOLs were subjected to immunostaining experiments before and after differentiation culture as following steps:
5 × 10⁴ fibroblasts were planted on coated glass coverslips on the day before induction. The induction was carried out in the same manner as in Example 1. After induction, the obtained IPOCs were fixed for 20 min at room temperature in 4% paraformaldehyde in PBS, and then permeabilized for 30 min in PBS containing 0.2% Triton X-100 and 10% goat serum (NGS), and incubated overnight at 4 °C in PBS containing 10% NGS and primary antibodies (mouse anti-O4 (Millipore, 1:50), rabbit anti-NF (Sigma-Aldrich, 1:1000), mouse anti-A2B5 (Millipore, 1:50), MBP (1:100, Covance; 1:100, Abcam), MAG (Millipore, 1:50), MOG (Millipore, 1:50). Then cells were washed three times with PBS and incubated for 2 h at room temperature with anti-rabbit or anti-mouse secondary antibodies Alexa Fluor-488 or Alexa Fluor-594 (1:500, Invitrogen). The cells were obtained by using immunofluorescence microscope or Zeiss LSM 510 META confocal microscope. The results were shown in Fig.2, where Merge is the coincidence map. Fig.2A showed the immunostaining results of iOPCs before differentiation culture, while Fig.2B showed the immunostaining results of iOLs after differentiation culture.

The result in Fig.2A showed that about 80% of fibroblasts were converted to O4-positive cells with characteristic morphology of oligodendrocyte progenitor cells (OPC). Furthermore, these O4 positive cells also showed positive staining with two additional OPC markers, A2B5 (oligodendrocyte precursor cell surface antigen A2B5) and S100β (glial specific protein). In contrast, after 4 weeks in the same induction medium without ROCK inhibitor, cells kept fibroblast morphology with negative staining of O4 (oligodendrocyte precursor cell surface antigen), A2B5, and S100β. These results suggest that ROCK inhibitor leads to direct conversion of human fibroblasts to iOPCs. The results of Fig.2B showed that about 70% of the iOPCs differentiated into cells with a multiprocessed morphology typical of oligodendrocytes within 3-7 days). The oligodendrocytes expressed myelin basic protein (MBP), and other specific markers of mature oligodendrocytes, including myelin-associated glycoprotein (MAG) and myelin oligodendrocyte glycoprotein (MOG). Thus, it is concluded that ROCK inhibitor-induced iOPCs can be further differentiated into mature functional oligodendrocytes.

### EXAMPLE 4:

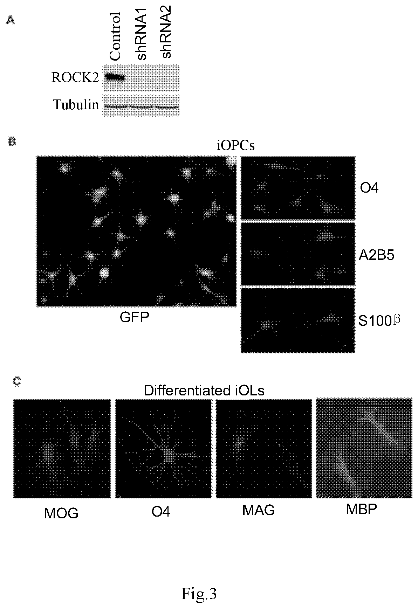

ROCK is a kinase belonging to the AGC (PKA/ PKG/PKC) family of serine-threonine kinases, including ROCK1 and ROCK2 in mammals (human, rat, mouse). ROCK1 is mainly expressed in the lung, liver, spleen, kidney and testis, while ROCK2 is distributed mostly in the brain and heart. To determine if the Rho-ROCK pathway is involved in fibroblast-OPC conversion, inventors examined the effects of ROCK knockdown on fibroblast-OPC conversion as follows:
Lentiviral constructs expressing short hairpin RNAs including shRNA1 and shRNA2 (Sigma-Aldrich, TRCN0000342532, TRCN0000342473) against ROCK2 gene were provided. And shRNA1 and shRNA2 were transfected to human adult dermal fibroblasts, respectively. And the transfected cells were subjected to western blot analysis. Next, the transfected cells were cultured with a neutral-inducing medium without a protein kinase inhibitor for 7 days to obtain iOPCs, and then the iOPCs were subjected to immunostaining experiments using the same procedure as in Example 3, followed by differentiated culturing the obtained iOPCs by using a mature medium to obtain iOLs. The obtained iOLs then were subjected to immunostaining experiments in the same manner as in Example 3. The results are shown in Fig. 3. Fig. 3A is the result of Western blot analysis, where Tubulin is the tubulin, and Control is the control group. Fig. 3B is the result of iOPCs immunostaining test, and Fig. 3C is the result of iOLs immunostaining test.

Fig. 3A showed that transfected cells showed significantly reduced ROCK2 expression, indicating that shRNA1 and shRNA2 are effective in inhibiting the expression of ROCK2. After culturing in neural-inducing medium, the transfected cells gave a conversion rate (60-80%) essentially same to that of ROCK inhibitor treatment. Fig.3B showed that the iOPCs induced by ROCK2 knockdown showed positive staining for OPC markers O4, A2B5 and S100β. Moreover, Fig.3C showed that these iOPCs obtained by ROCK2 gene silencing can differentiate into cells with typical oligodendrocytes morphology after differentiation culture, and the differentiated cells (iOLs) expressed MBP, MAG, and MOG (Fig. 3c). The results dindicated that ROCK2 kinase is involved in conversion of fibroblast to neural cells.

### EXAMPLE 5:

Inventors hypothesize that ROCK inhibitor induces fibroblast-OPC conversion by regulating a set of neural transcription factors. To analyze the similarities among iOPCs obtained with Y27632 treatment, human brain-derived OPCs, iOLs and parental fibroblasts, inventors generated comparative global gene expression data by microarray analysis. Microarray analysis was performed at the Washington University Genome Center. Specific steps are as follows, Illumina HumanHT-12 v4 Expression BeadChip was used and samples from above cells were labeled by biotin. The Direct Hybridization Assay was performed and the data were scanned on the BeadArray Reader. Scanned images were quantitated by Illumina Beadscan, v3. Quantitative data was imported into Illumina GenomeStudio software and normalized by Illumina's quantile method. The quantile-normalized background-subtracted data were calculated in excel. The data were filtered based on the average signal, and set the baseline as 50 so that only the genes of which average signal >50 could be used for further analysis. To each gene, maximum and minimum signal values were selected, and then divided between them. Only the genes of the division value >3 were selected and considered as differently expressed. All of the differently expressed genes were clustered by using MeV software.

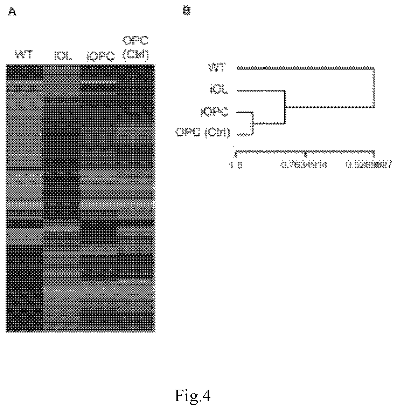

To determine the relationship among fibroblasts and iOPC, brain-derived OPC and iOL cells, microarray analysis was performed on each sample separately. The obtained data were filtered based on average signal and baseline was set as 50 to increase the sensitivity. To examine the potential OPC differentiation pathways relevant to the immortalization, all OPC differentiation genes from Gene Ontology Website (http://www.geneontology.org/, GO: 0030182) were compared with the microarray data. For gene enrichment analysis, inventors used web-based Gorilla program (http://cbl-gorilla.cs.technion.ac.il/).The test results are shown in Fig.4, where WT represents wild type. Fig.4A shows the gene differential expression profiles between iOPCs, human brain-derived OPCs, iOLs and parental fibroblasts, and Fig.4B shows the results of cluster analysis of differentially expressed genes between iOPCs, human brain-derived OPCs, iOLs and parental fibroblasts.

As can be seen from Fig. 4, cluster analysis revealed a significant difference of gene expression profiles between iOPC cells and their parental fibroblasts (Fig. 4A), suggesting that iOPCs are clearly distinct from parental fibroblasts. Clustering analysis of differential gene expression revealed that the transcription profile of ROCK inhibitor-induced iOPCs were tightly clustered with the transcription profile of human brain-derived OPCs and were distant from the profile of the parental fibroblasts (Figs. 4A and B). By analysis of the microarray data of the genes of 4-folds differential expression, iOPCs and brain-derived OPCs showed significant gene expression overlap for neural transcription factors. These above data suggest that there may be permanent down-regulation of a set of fibroblast specific genes during induced conversion. Taken together, these findings indicate that the genetic transfer-differentiation erased the majority of the evident expression hallmarks of the cell of origin, whereas specifically inducing the OPC phenotypic expression.

### EXAMPLE 6:

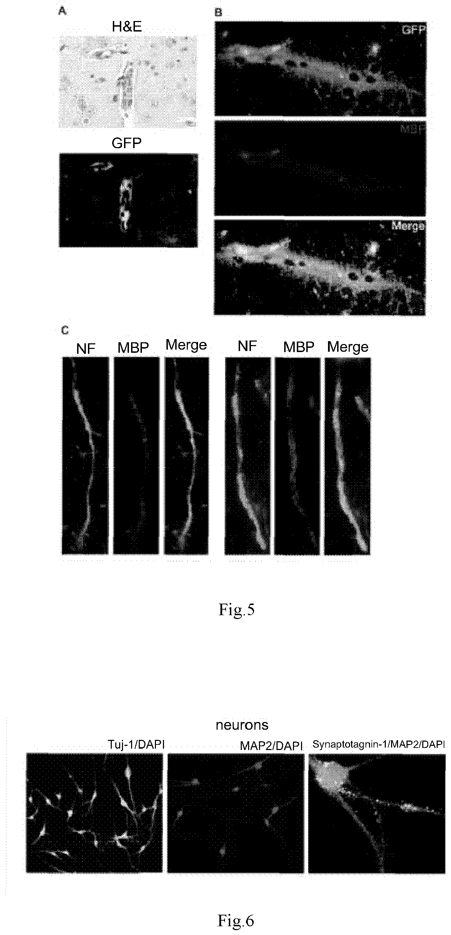

The iOPCs obtained by the ROCK inhibitor Y27632 in Example 1 were transplanted into the demyelinating brain corpus callosum of mice treated with cuprizone to evaluate the functionality of iOPCs in vivo and the potential to form myelin as follows:
C57BL/6 mice were put on a diet of 0.2% (w/w) cuprizone (Sigma Aldrich), a copper chelator. Feeding cuprizone diet for 12 weeks results in depletion of the pool of endogenous OPCs in the corpus callosum and finally leads to its complete demyelination. At 12 weeks after the start of the cuprizone diet, mice were divided into three groups. In the first group, 4 µl of PBS solution containing iOPCs induced by ROCK inhibitor was injected into the right side of the brain corpus callosum of cuprizone-mice (n = 6, left as untransfected control group, where n represents the number of mice). In the second group, 4 µl of PBS solution was injected into the right side of the brain corpus callosum of cuprizone-mice (n = 3, left as untransfected control group, where n represents the number of mice). In the third group, 4 µl of PBS solution containing parental fibroblasts was injected into the right side of the brain corpus callosum of cuprizone-mice (n = 3, left as untransfected control group, where n represents the number of mice). The concentration of the injected cells is 25,000 cells / µl. The stereotactic co-ordinates of the injection position are 10.98 mm (anteroposterior axis), 21.75 mm (lateromedial axis), 22.25mm (vertical axis) (Copray et al., 2006; Sher et al.,2009). After the implantation, mice were taken off the cuprizone diet and put back on normal diet to avoid degeneration of the implanted cells by cuprizone. After 4 weeks of transplantation, the mice were sacrificed and the brain was resected. Then, Immunohistochemical staining (MBP staining) was performed on the brain tissue sections. The results are shown in Fig.5, where NF represents the neurofilament protein. Fig.5A are images of the immunofluorescence results and H & E staining results for the site of iOPs injection of the same brain tissue sections. Figure 5B are images of the immunostaining results for the site of iOPCs injection of brain tissue sections. Figure 5C are confocal microscopic photographs of the iOPCs site of injection of the brain tissue sections.

Fig.5A shows that only about 20% of the implanted cells survived. Fig.5B shows that the surviving iOPCs developed into mature MBP-expressing OLs that contributed to the remyelination of the corpus callosum axons. Confocal microscopy in Fig.5C revealed that MBP+ tube-like structures and the nerve fibers co-coat the structure of the nerve fibers, indicating the implanted iOPCs promote the host axon to produce myelin. No similar structures were observed in nontransplanted brains. These results demonstrated that iOPCs induced by ROCK inhibitors can be transformed into oligodendrocytes that can produce myelin in vivo.

### EXAMPLE 7:

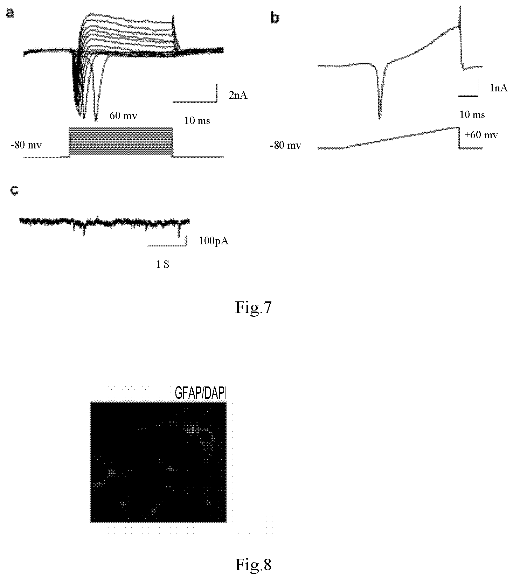

To assess the general application of ROCK inhibitor for direct conversion of different neural cells, inventors tweaked the protocol for induction of iOPCs using the following procedure:
Coat tissue culture 24-well plate with 0.1 mg/ml Poly-L-ornithine solution (sigma, P4957) for at least 3 hours; wash wells 3 times with autoclaved MilliQ water, 5 mins each; add 500ul/well of 2 µg/ml Fibronectin (sigma, F0556) and 10 µg/ml Laminin (ROCHE, 11243217001) solution in IX PBS and place in incubator overnight to coat. Aspirate the solution from the well, wash with basic medium and plate fibroblast cells (ATCC, PCS-201-012) with basic medium at 2x10^4 cells per well, and culture with the above basic medium for 24 hours. Aspirate basic medium from the well, wash with PBS once, and feed cells with induction medium containing 5 µM ROCK inhibitor Y27632 and 2 µM mTOR inhibitor P529. After culturing for 3-7 days, cells change their morphology into neural-like cells, and then culture the obtained neural-like cells with mature medium or OPC medium, and induced neuronal cells (iNCs) and astrocytes were obtained. Furthermore, immunostaining experiments and patch-clamp experiments were performed on the induced neurons (iNCs) and astrocytes. The results of the immunostaining assay of iNCs induced by Y27632 and mTOR inhibitors are shown in Figure 6. The results of the patch-clamp experiment of iNCs induced by Y27632 and mTOR inhibitors are shown in Figure 7. Fig.7a shows current traces (upper panel) recorded in the process in which cells were depolarized by voltage steps from -60 to +60 mV in 10 mV increments and the relationship (lower panel) between the current-voltage (I-V) and sodium current. Fig.7b shows traces of membrane currents recorded in the process of a voltage ramp from -80mV to +60mV over 500ms. Fig.7c shows traces of spontaneous synaptic currentsrecorded at a holding potential of -80 mV. The the immunostaining results of astrocyte cells induced by Y27632and mTOR inhibitors were shown in Fig.8.

From the results of the above experiments, the inventors succeeded in converting fibroblasts into typical neural cells and astrocytes. Neural cells and astrocytes induced by Y27632 and mTOR inhibitors showed typical cell types morphology and biomarkers. For example, it can be seen from Fig.6 that the induced neural cells (iNCs) express neural specific markers Tuj (microtubule associated protein), MAP2 (microtubule associated protein 2) and Synaptotagnin (synaptic binding protein) The results of Fig.7 show that the induced neural cells exhibit typical electrophysiological properties of neural cells. As can be seen from Fig.8, the resulting astrocytes are induced to express astrocyte-specific markers GFAP (Glial fibrillary acidic protein). The above results indicate that protein kinase inhibitors are capable of inducing various neural cells, such as neuronal cells, astrocytes, etc., from non-neural cells.

## Claims

1. A method for preparing a oligodendrocyte progenitor cell, comprising:
culturing a fibroblast cell with a first medium,
wherein the first medium consists of:
a first basic medium,
a first protein kinase inhibitor,
BDNF,
NT3,
VPA,
dbcAMP, and
Retinoic acid,
wherein the first protein kinase inhibitor is at least one selected from the group consisting of Y27632, Palomid 529, LY294002, PF562271 and rapamycin.

2. The method of claim 1, further comprising:
culturing the cell with a second medium or a third medium after the culturing with the first medium,
wherein the second medium comprises:
a second basic medium,
a second protein kinase inhibitor,
N-2,
B-27 without vitamin A,
Glutamax,
SHH,
FGF2,
PDGF-AA, and
Retinoic acid,
and
the third medium comprises:
a third basic medium,
a third protein kinase inhibitor,
N-2,
B-27 without vitamin A,
Glutamax,
SHH,
Noggin,
dbcAMP,
IGF,
NT3, and
Retinoic acid,
wherein the first basic medium is neuronal medium, the second basic medium is DMEM/F12 medium, the third basic medium is DMEM/F12 medium,
preferably the second protein kinase inhibitor is at least one selected from the group consisting ofY27632, Palomid 529, LY294002, PF562271 and rapamycin, and
the third protein kinase inhibitor is at least one selected from the group consisting of Y27632, Palomid 529, LY294002, PF562271 and rapamycin.

3. The method of claim 2, wherein the first medium comprises:
5µM - 20µM ROCK inhibitor (Y27632),
5 ng/ml - 20 ng/ml BDNF,
5 ng/ml - 20 ng/ml NT3,
0.5 mM- 1.5 mM VPA,
25µM -100 µM dbcAMP, and
0.5µM - 1µM retinoic acid;
the second medium comprises
5µM - 20µM Y27632,
1x N-2,
1x B-27 without vitamin A,
1x Glutamax,
100 ng/ml - 400 ng/ml SHH,
10 ng/ml - 40 ng/ml FGF2,
10 ng/ml - 40 ng/ml PDGF-AA, and
0.5µM - 1µM retinoic acid; and
the third medium comprises:
5µM - 20µM Y27632,
1x N-2,
1x B-27 without vitamin A,
1x Glutamax,
100 ng/ml - 400 ng/ml SHH,
50 ng/ml - 200 ng/ml Noggin,
50 ng/ml - 200 ng/ml dbcAMP,
50 ng/ml - 200 ng/ml IGF,
5 ng/ml - 20 ng/ml NT3, and
0.5µM - 1µM retinoic acid

4. The method of claim 3, wherein the first medium comprises:
10µM Y27632,
10 ng/ml BDNF,
10 ng/ml NT3,
1 mM VPA,
50 µM dbcAMP, and
0.5µM retinoic acid;
the second medium comprises:
10µM Y27632,
1x N-2,
1x B-27 without vitamin A,
1x Glutamax,
200 ng/ml SHH,
20 ng/ml FGF2,
20 ng/ml PDGF-AA, and
0.5µM retinoic acid;
the third medium comprises:
10µM Y27632,
1x N-2,
1x B-27 without vitamin A,
1x Glutamax,
200 ng/ml SHH,
100 ng/ml Noggin,
100 ng/ml dbcAMP,
100 ng/ml IGF,
10 ng/ml NT3, and
0.5µM retinoic acid.

## Patentansprüche

1. Eine Methode zur Herstellung einer Oligodendrozyten-Vorläuferzelle, umfassend:
Kultivieren eine Fibroblastenzelle mit einem ersten Medium,
wobei das erste Medium besteht aus:
einem ersten Grundmedium,
einem ersten Proteinkinaseinhibitor,
BDNF,
NT3,
VPA,
dbcAMP, und
Retinsäure,
wobei der erste Proteinkinaseinhibitor mindestens einer ausgewählt aus der Gruppe bestehend aus Y27632, Palomid 529, LY294002, PF562271 und Rapamycin ist.

2. Die Methode von Anspruch 1, weiterhin umfassend:
Kultivieren der Zelle mit einem zweiten Medium oder einem dritten Medium nach dem Kultivieren mit dem ersten Medium,
wobei das zweite Medium umfasst:
ein zweites Grundmedium,
einen zweiten Proteinkinaseinhibitor,
N-2,
B-27 ohne Vitamin A,
Glutamax,
SHH,
FGF2,
PDGF-AA, und
Retinsäure,
und
das dritte Medium umfasst:
ein drittes Grundmedium,
einen dritten Proteinkinaseinhibitor,
N-2,
B-27 ohne Vitamin A,
Glutamax,
SHH,
Noggin,
dbcAMP,
IGF,
NT-3, und
Retinsäure,
wobei das erste Grundmedium Neuronal-Medium ist, das zweite Grundmedium DMEM/F12-Medium ist, das dritte Grundmedium DMEM/F12-Medium ist,
vorzugsweise ist der zweite Proteinkinaseinhibitor mindestens einer ausgewählt aus der Gruppe bestehend aus Y27632, Palomid 529, LY294002, PF562271 und Rapamycin, und
der dritte Proteinkinaseinhibitor ist mindestens einer ausgewählt aus der Gruppe bestehend aus Y27632, Palomid 529, LY294002, PF562271 und Rapamycin.

3. Die Methode von Anspruch 2, wobei das erste Medium umfasst:
5µM - 20µM ROCK inhibitor (Y27632),
5 ng/ml - 20 ng/ml BDNF,
5 ng/ml - 20 ng/ml NT3,
0.5 mM- 1.5 mM VPA,
25µM -100 µM dbcAMP, und
0.5µM - 1µM Retinsäure;
das zweite Medium umfasst:
5µM - 20µM Y27632,
1x N-2,
1x B-27 ohne Vitamin A,
1x Glutamax,
100 ng/ml - 400 ng/ml SHH,
10 ng/ml - 40 ng/ml FGF2,
10 ng/ml - 40 ng/ml PDGF-AA, und
0.5µM - 1µM Retinsäure; und
das dritte Medium umfasst:
5µM - 20µM Y27632,
1x N-2,
1x B-27 ohne Vitamin A,
1x Glutamax,
100 ng/ml - 400 ng/ml SHH,
50 ng/ml - 200 ng/ml Noggin,
50 ng/ml - 200 ng/ml dbcAMP,
50 ng/ml - 200 ng/ml IGF,
5 ng/ml - 20 ng/ml NT3, und
0.5µM - 1µM Retinsäure

4. Die Methode von Anspruch 3, wobei das erste Medium umfasst:
10µM Y27632,
10 ng/ml BDNF,
10 ng/ml NT3,
1 mM VPA,
50 µM dbcAMP, und
0.5µM Retinsäure;
das zweite Medium umfasst:
10µM Y27632,
1x N-2,
1x B-27 ohne Vitamin A,
1x Glutamax,
200 ng/ml SHH,
20 ng/ml FGF2,
20 ng/ml PDGF-AA, und
0.5µM Retinsäure;
das dritte Medium umfasst:
10µM Y27632,
1x N-2,
1x B-27 ohne Vitamin A,
1x Glutamax,
200 ng/ml SHH,
100 ng/ml Noggin,
100 ng/ml dbcAMP,
100 ng/ml IGF,
10 ng/ml NT3, und
0.5µM Retinsäure.

## Revendications

1. Procédé pour la préparation d'une cellule progénitrice d'oligodendrocyte, comprenant:
la culture d'une cellule de type fibroplaste avec un premier milieu,
le premier milieu étant constitué de:
un premier milieu alcalin,
un premier inhibiteur de protéine kinase,
BDNF,
NT3,
VPA,
dbcAMP, et
de l'acide rétinoïque,
le premier inhibiteur de protéine kinase étant au moins un inhibiteur choisi dans le groupe constitué par le Y27632, le palomid 529, le LY294002, le PF562271 et la rapamycine.

2. Procédé selon la revendication 1, comprenant en outre:
la culture de la cellule avec un deuxième milieu ou un troisième milieu après la culture avec le premier milieu,
le deuxième milieu comprenant:
un deuxième milieu alcalin,
un deuxième inhibiteur de protéine kinase,
N-2,
B-27 sans vitamine A,
Glutamax,
SHH,
FGF2,
PDGF-AA, et
de l'acide rétinoïque,
et
le troisième milieu comprenant:
un troisième milieu alcalin,
un troisième inhibiteur de protéine kinase,
N-2,
B-27 sans vitamine A,
Glutamax,
SHH,
Noggin,
dbcAMP,
IGF,
NT3, et
de l'acide rétinoïque,
le premier milieu alcalin étant un milieu neuronal, le deuxième milieu alcalin étant un milieu DMEM/F12, le troisième milieu alcalin étant un milieu DMEM/F12,
préférablement le deuxième inhibiteur de protéine kinase étant au moins un inhibiteur choisi dans le groupe constitué par le Y27632, le palomid 529, le LY294002, le PF562271 et la rapamycine, et
le troisième inhibiteur de protéine kinase étant au moins un inhibiteur choisi dans le groupe constitué par le Y27632, le palomid 529, le LY294002, le PF562271 et la rapamycine.

3. Procédé selon la revendication 2, le premier milieu comprenant:
5 µM à 20 µM en inhibiteur de ROCK (Y27632),
5 ng/ml à 20 ng/ml de BDNF,
5 ng/ml à 20 ng/ml de NT3,
0,5 mM à 1,5 mM en VPA,
25 µM à 100 µM en dbcAMP, et
0,5 µM à 1 µM en acide rétinoïque;
le deuxième milieu comprenant
5 µM à 20 µM en Y27632,
1 x N-2,
1 x B-27 sans vitamine A,
1 x Glutamax,
100 ng/ml à 400 ng/ml de SHH,
10 ng/ml à 40 ng/ml de FGF2,
10 ng/ml à 40 ng/ml de PDGF-AA, et
0,5 µM à 1 µM en acide rétinoïque; et
le troisième milieu comprenant:
5 µM à 20 µM en Y27632,
1 x N-2,
1 x B-27 sans vitamine A,
1 x Glutamax,
100 ng/ml à 400 ng/ml de SHH,
50 ng/ml à 200 ng/ml de Noggin,
50 ng/ml à 200 ng/ml de dbcAMP,
50 ng/ml à 200 ng/ml d'IGF,
5 ng/ml à 20 ng/ml de NT3, et
0,5 µM à 1 µM en acide rétinoïque.

4. Procédé selon la revendication 3, le premier milieu comprenant:
10 µM en Y27632,
10 ng/ml de BDNF,
10 ng/ml de NT3,
1 mM en VPA,
50 µM en dbcAMP, et
0,5 µM en acide rétinoïque;
le deuxième milieu comprenant:
10 µM en Y27632,
1 x N-2,
1 x B-27 sans vitamine A,
1 x Glutamax,
200 ng/ml de SHH,
20 ng/ml de FGF2,
20 ng/ml de PDGF-AA, et
0,5 µM en acide rétinoïque;
le troisième milieu comprenant:
10 µM en Y27632,
1 x N-2,
1 x B-27 sans vitamine A,
1 x Glutamax,
200 ng/ml de SHH,
100 ng/ml de Noggin,
100 ng/ml de dbcAMP,
100 ng/ml d'IGF,
10 ng/ml de NT3, et
0,5 µM en acide rétinoïque.
